# EUROPEAN PATENT SPECIFICATION

(11) **EP 0 498 069 B1**
(45) Date of publication and mention of the grant of the patent: **25.10.1995**
(21) Application number: 91121403.9
(22) Date of filing: 13.12.1991
(51) Int. Cl.: A61K 38/12

(54) **New use of peptide derivative**
Neue Verwendung von Peptidderivat
Nouvelle utilization d'un dérivé de peptide

(30) Priority: 21.12.1990 JP 418298/90
(43) Date of publication of application: 12.08.1992
(73) Proprietor: FUJISAWA PHARMACEUTICAL CO., LTD., Osaka-shi Osaka 541 (JP)
(72) Inventor: Fujii, Takashi, Ikeda-shi, Osaka 563 (JP); Tomoi, Masaaki, Higashiosaka-shi, Osaka 578 (JP)
(74) Representative: Türk, Gille, Hrabal, Leifert

(56) References cited:
- EP-A- 394 989
- EP-A- 0 336 230
- EP-A- 0 400 637

## Description

This invention relates to a new use of known peptide derivatives, in particular it relates to the use of peptide derivatives of general formula (I) following below or pharmaceutical acceptable salts thereof for the manufacture of a medicament for preventing and/or treating inflammation, especially arthritis.

Peptide derivatives of general formula (I) following below are already known from EP-A-0 336 230. It is also known from this reference that peptide derivatives of formula (I) possess pharmacological activities such as substance P antagonism and neurokinin A antagonism and are useful in the preparation of medicaments for use in the treatment and prevention of asthma.

From EP-A-0 394 989 peptide compounds having a quite different basic structure are known which are useful in the preparation of medicaments for use in the treatment of inflammatory diseases such as rheumatoid arthritis. Furthermore, these peptide compounds are said to be antagonists of substance P and neurokinins A and B and therefore useful in the treatment or prevention of tachykinin mediated diseases, such as inflammation.

It is the object of the present invention to find compounds which are more useful for treating or preventing inflammatory diseases as those disclosed in EP-A-0 394 989.

Surprisingly, the applicant has found that the peptide derivatives of general formula (I) as disclosed in EP-A-0 336 230 have a strong antiinflammatory acitivity and therefore can be used for the manufacture of a medicament for preventing and/or treating inflammation, in particular arthritis.

Subject-matter of the present invention is the use of a peptide derivative of the following general formula 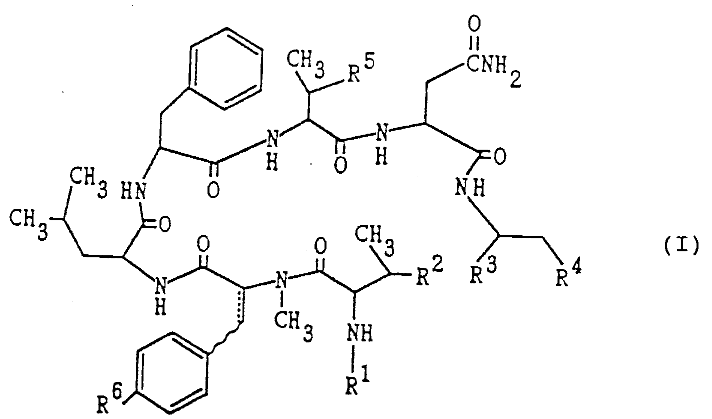 wherein
R¹ is hydrogen, phenyl(Cᵢ-C₆)alkoxycarbonyl, Ci-C₆-alkanoyl, C₁₅-C₂₀-alkanoyl, benzoyl, thienyl(C₁-C₆)alkanoyl, phenyl(C₃-C₆)alkenoyl substituted with a C₂-C₆-alkenyl group, or phenyl(C₁-C₆)alkanoyl substituted with a C₁-C₆-alkyl group;
_{R}² is hydroxy and
R³ is carboxy or C1-C6-alkoxycarbonyl, or
R² and R³ are linked together to represent a group of the formula: 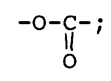
R⁴ is hydroxy, phenyl(C₁-C₆)alkoxy or C₁-C₆-alkanoyloxy;
R⁵ is hydroxy, phenyl(C₁-C₆)alkoxy or C₁-C₆-alkanoyloxy;
R⁶ is hydroxy, C₁-C₆-alkoxy, phenyl(C₁-C₆)alkoxy or C₁-C₆-alkanoyloxy; and is a single bond or a double bond,
   or a pharmaceutically acceptable salt thereof for the manufacture of a medicament for preventing and/or treating inflammation.

The peptide derivatives of the above general formula (I) used according to the present invention possess a strong antiinflammatory activity as can be seen from the test results summarized in the table at page 8 following below.

According to preferred embodiments the present invention relates to the use of a peptide derivative of general formula (I), wherein:
R¹ is 3-(2-pentylphenyl)propanoyl,

R² and R³ are linked together to represent a group of the formula : 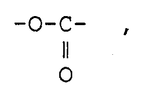
_{R}⁴ is hydroxy,
R⁵ is hydroxy,
_{R}⁶ is hydroxy, and is a double bond,
or a pharmaceutically acceptable salt thereof ,
   especially the compound 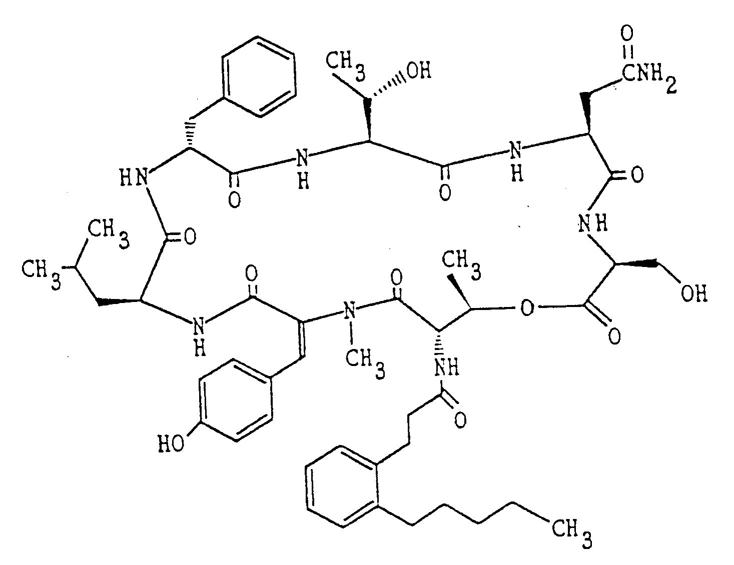 According to a further preferred embodiment the present invention relates to the use of a peptide derivative of general formula (I) as defined above or a pharmaceutical acceptable salt thereof for the manufacture of a medicament for preventing and/or treating arthritis.

Suitable pharmaceutically acceptable salts of the compound (I) are conventional non-toxic salts and may include a salt with a base or an acid addition salt such as a salt with an inorganic base, for example an alkali metal salt (e.g. lithium salt, sodium salt, and potassium salt), an alkaline earth metal salt (e.g. calcium salt, and magnesium salt), an ammonium salt; a salt with an organic base, for example, an organic amine salt (e.g. triethylamine salt, pyridine salt, picoline salt, ethanolamine salt, triethanolamine salt, dicyclohexylamine salt, and N,N'-dibenzylethylenediamine salt); an inorganic acid addition salt (e.g. hydrochloride, hydrobromide, sulfate, and phosphate); an organic carboxylic or sulfonic acid addition salt (e.g. formate, acetate, trifluoroacetate, maleate, tartrate, methanesulfonate, benzenesulfonate, and p-toluenesulfonate); a salt with a basic or acidic amino acid (e.g. arginine, aspartic acid, and glutamic acid).

The compound (I) or a pharmaceutically acceptable salt thereof, used in accordance with the invention, has antiinflammatory activities and, as such, is of value as an active ingredient for use in antiinflammatory compositions. These antiinflammatory compositions are effective in preventing and treating inflammation such as various types of arthritis [e.g. articular rheumatism (e.g. rheumatoid arthritis), osteoarthrosis (e.g. osteoarthrosis of knee), periarthritis humeroscapularis, cervico-omo-brachial syndrome] or the like.

The compound (I) or a pharmaceutically acceptable salt thereof, which is used as an active ingredient of said compositions, can be administered as it is but is generally administered in the form of a pharmaceutically acceptable preparation.

The pharmaceutical preparation mentioned above may take a variety of forms such as oral preparation [e.g. solution, emulsion, suspension, capsule, granule, powder, tablet, and syrup], injection, suppository or the like.

Such preparations can be manufactured by the established pharmaceutical procedures using appropriate excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrators such as starch, carboxymethylcellulose, hydroxypropyl starch, sodium bicarbonate, calcium phosphate, and calcium citrate; lubricants such as magnesium stearate, talc, and sodium laurylsulfate; corrigents such as citric acid, menthol, glycine, and orange powder; preservatives such as sodium benzoate, sodium bisulfite, benzyl alcohol, methylparaben, and propylparaben, stabilizers such as citric acid, sodium citrate, and acetic acid; suspending agents such as methylcellulose, polyvinylpyrrolidone, and aluminum stearate; dispersants such as hydroxypropylmethylcellulose, polysorbate 80, and carboxymethylcellulose; solvents such as water; isotonicity such as sodium chloride; base waxes such as cacao butter, polyethylene glycol, and white soft paraffine; and so on.

The dosage for the pharmaceutical composition of this invention depends on the patient's age, body weight and clinical condition, the method of administration and other conditions. Generally speaking, however, the active compound (I) or a pharmaceutically acceptable salt thereof is administered in doses of 1 to 300 mg and preferably 10 to 100 mg, either orally or parenterally (for examples intraarticularly, intravenously).

The following test example are intended to demonstrate the excellent antiinflammatory actions of the compound (I) or a pharmaceutically acceptable salt thereof used according to the present invention.

### Test compound

The compound of the following structural formula : 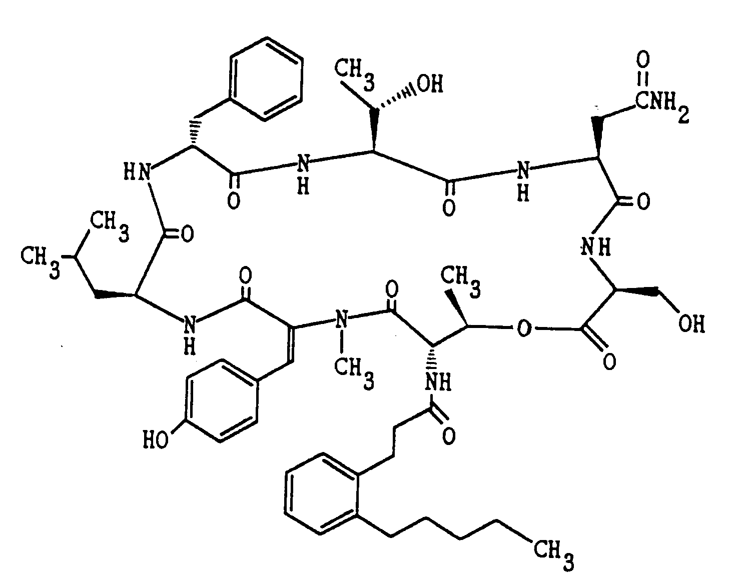 (hereinafter referred to briefly as tetrahydro-WS9326A)

### Effect on carrageenin-induced rat knee edema :

Eight-week-old male Wistar rats were used.

Each rat was anesthetized with 0.2 ml of pentobarbital sodium (50 mg/ml) in physiological saline i.p. and then intravenously dosed with 2 ml/kg of Evans blue (10 mg/ml) in physiological saline. Thereafter, 0.1 ml of 2% carrageenin in physiological saline was administered into the articular cavity of the right knee. After 4 hours, the animal was sacrificed, skinned to expose the articular region and observed for leakage of Evans blue. Then, physiological saline (0.15 ml) was injected into the articular cavity. After incision, 5 αl of synovial fluid was collected. The synovial fluid obtained was diluted with physiological saline (40 µl) and using a cell counter, the cells were counted under the microscope.

The test compound (tetrahydro-WS9326A) was suspended in 0.1% methylcellulose in physiological saline and 0.1 ml of this suspension was administered into the knee joint 15 minutes before carrageenin administration.

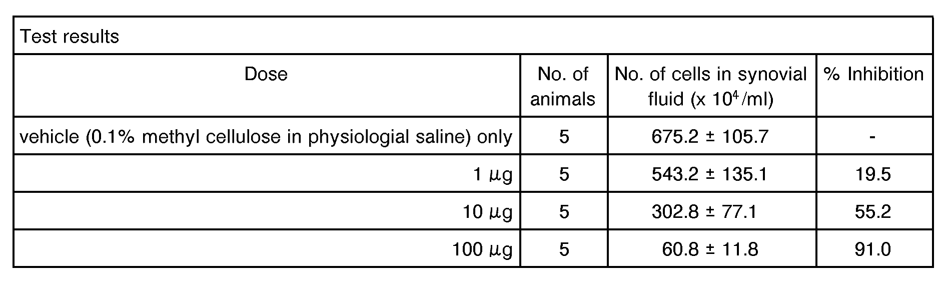

### Toxicity study

An injectable suspension containing the test compound and other ingredients mentioned below was administered into the knee joint of rabbits (6 males and 6 females) and dogs (3 males and 3 females) twice a week for 4 weeks. The dose was 8 mg/kg for rabbits and 12 mg/kg for dogs. The administration of the test compound caused death in neither rabbits nor dogs.

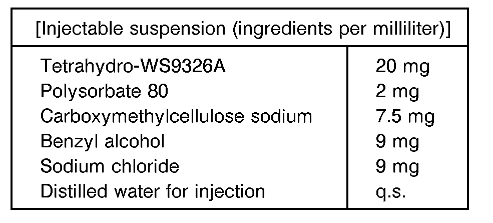

The following examples are illustrative of the invention.

### Example 1

A powder of the following composition is encapsulated to provide capsules.

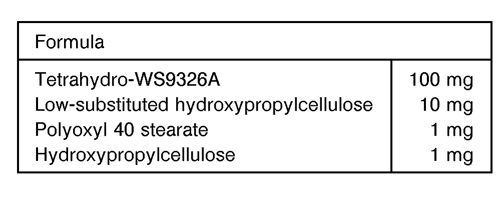

### Example 2

The following ingredients are admixed and granulated in the routine manner to provide granules.

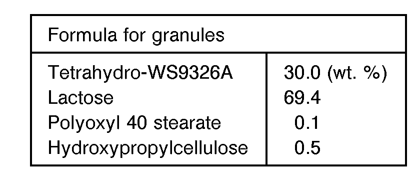

### Example 3

The following ingredients are admixed and powdered in the routine manner to provide a powder.

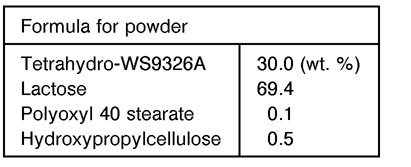

### Example 4

The following ingredients are admixed and compressed in the routine manner to provide tablets.

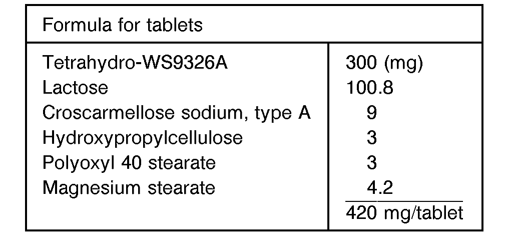

Where necessary, the tablets thus obtained are film-coated or enteric-coated to provide film coated or enteric coated tablets.

### Example 5

To 100 ml of distilled water for injection is added 1 g of carboxymethylcellulose sodium and the mixture is stirred. To the resulting solution is added 40 mg of Polysorbate 80 and, after mixing, 1 g of tetrahydro-WS9326A is dispersed therein to provide an injectable suspension.

### Example 6

To 50 ml of macrogol 400 is added 1 g of tetrahydro-WS9326A and the mixture is stirred. The resulting solution is made up to 100 ml with distilled water for injection to provide an injectable solution.

### Example 7

After tetrahydro-WS9326A is dispersed in a solution of Polysorbate 80 in distilled water for injection, a solution of sodium chloride, benzyl alcohol and carboxymethylcellulose sodium in distilled water for injection is added thereto. 5 MI of the resulting suspension is filled into an ampoule, which is then sealed by fusion to provide an injectable suspension containing the following ingredients.

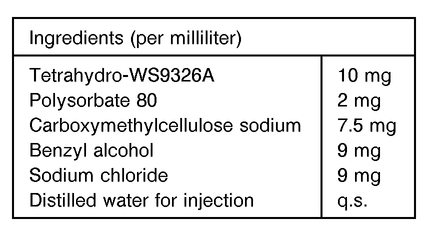

Reference

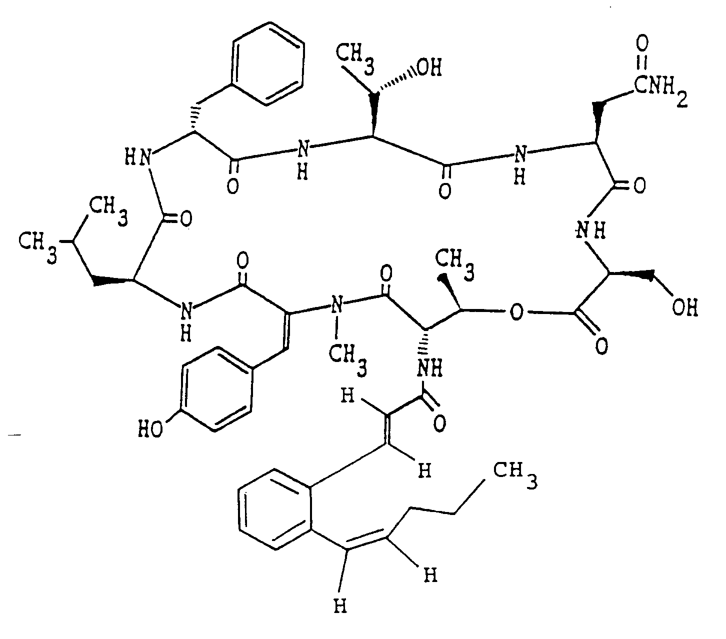

Tetrahydro-WS9326A was obtained by subjecting the compound of the above structural formula to reduction with 10% Pd-C.

## Claims

1. Use of a peptide derivative of the general formula : 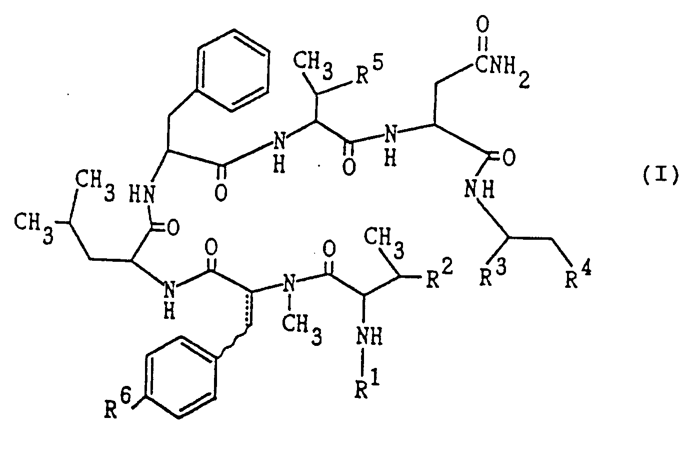 wherein
R¹ is hydrogen, phenyl(C₁-C₆)alkoxycarbonyl, C₁-C₆-alkanoyl, C₁₅-C₂₀-alkanoyl, benzoyl, thienyl(C₁-C₆)alkanoyl, phenyl(C₃-C₆)alkenoyl substituted with a C₂-C₆-alkenyl group, or phenyl(C₁-C₆)alkanoyl substituted with a C₁-C₆-alkyl group;
_{R}² is hydroxy and
R³ is carboxy or C₁-C₆-alkoxycarbonyl, or
R² and R³ are linked together to represent a group of the formula: 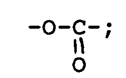
R⁴ is hydroxy, phenyl(C₁-C₆)alkoxy or C₁-C₆-alkanoyloxy;
R⁵ is hydroxy, phenyl(C₁-C₆)alkoxy or C₁-C₆-alkanoyloxy;
R⁶ is hydroxy, C₁-C₆-alkoxy, phenyl(C₁-C₆)alkoxy or C₁-C₆-alkanoyloxy; and -̅-̅-̅-̅-̅ is a single bond or a double bond,
or a pharmaceutically acceptable salt thereof for the manufacture of a medicament for preventing and/or treating inflammation.

2. Use of claim 1, in which the peptide derivative is the compound (I) wherein
R¹ is 3-(2-pentylphenyl)propanoyl,
R² and R³ are linked together to represent a group of the formula : 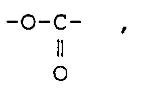
_{R}⁴ is hydroxy,
R⁵ is hydroxy,
_{R}⁶ is hydroxy, and -̅-̅-̅-̅-̅ is a double bond,
or a pharmaceutically acceptable salt thereof.

3. Use of claim 2, in which the peptide derivative is a compound of the formula:

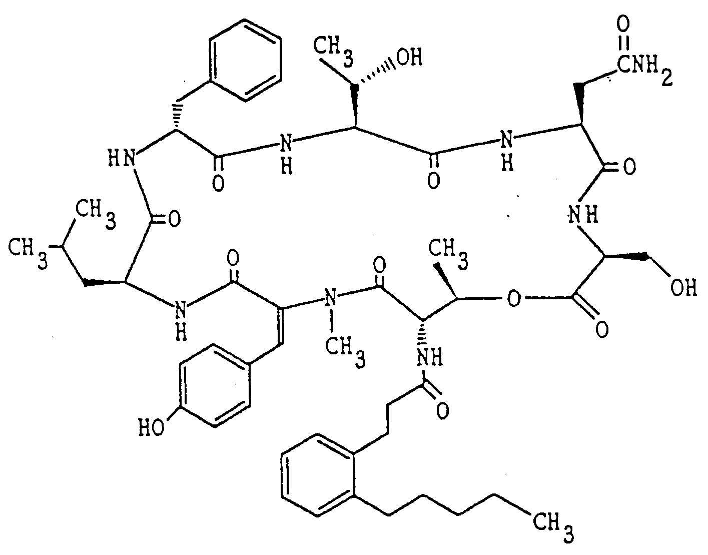

4. Use of a peptide derivative of claim 1 or a pharmaceutically acceptable salt thereof for the manufacture of a medicament for preventing and/or treating arthritis.

## Patentansprüche

1. Verwendung eines Peptidderivates der allgemeinen Formel: 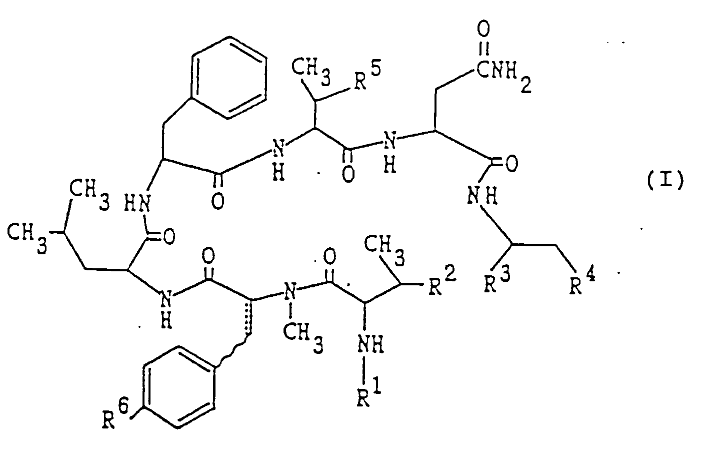 worin R¹ Wasserstoff, Phenyl(C₁-C₆)alkoxycarbonyl, (C₁-C₆)-Alkanoyl, (C₁₅-C₂₀)Alkanoyl, Benzoyl, Thienyl(C₁-C₆)alkanoyl, Phenyl(C₃-C₆)alkenyl, substituiert mit einer (C₂-C₆)-Alkenylgruppe, oder Phenyl(C₁-C₆)alkanoyl, substituiert mit einer (C₁-C₆)Alkylgruppe, ist;
R² Hydroxy ist und
R³ Carboxy oder (C₁ -C₆ )Alkoxycarbonyl ist, oder
R² und R³ miteinander verbunden sind, um eine Gruppe der Formel: 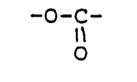 darzustellen;
R⁴ Hydroxy, Phenyl(C₁-C₆)alkoxy oder (C₁-C₆)Alkanoyloxy ist;
R⁵ Hydroxy, Phenyl(C₁-C₆)alkoxy oder (C₁-C₆)Alkanoyloxy ist;
R⁶ Hydroxy, (C₁-C₆))Alkoxy, Phenyl(C₁-C₆)alkoxy oder (C₁-C₆)Alkanoyloxy ist; und eine Einfachbindung oder eine Doppelbindung darstellt,
oder eines pharmazeutisch verträglichen Salzes davon zur Herstellung eines Medikamentes zur Prävention und/oder Behandlung von Entzündungen.

2. Verwendung nach Anspruch 1, worin das Peptidderivat die Verbindung (I), worin
R¹ 3-(2-Pentylphenyl)propanoyl ist,
R² und R³ miteinander verbunden sind, um eine Gruppe der Formel: 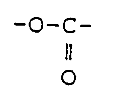 darzustellen,
R⁴ Hydroxy ist,
R⁵ Hydroxy ist,
R⁶ Hydroxy ist und
eine Doppelbindung ist,
oder ein pharmazeutisch verträgliches Salz davon ist.

3. Verwendung nach Anspruch 2, worin das Peptidderivat eine Verbindung der Formel ist:

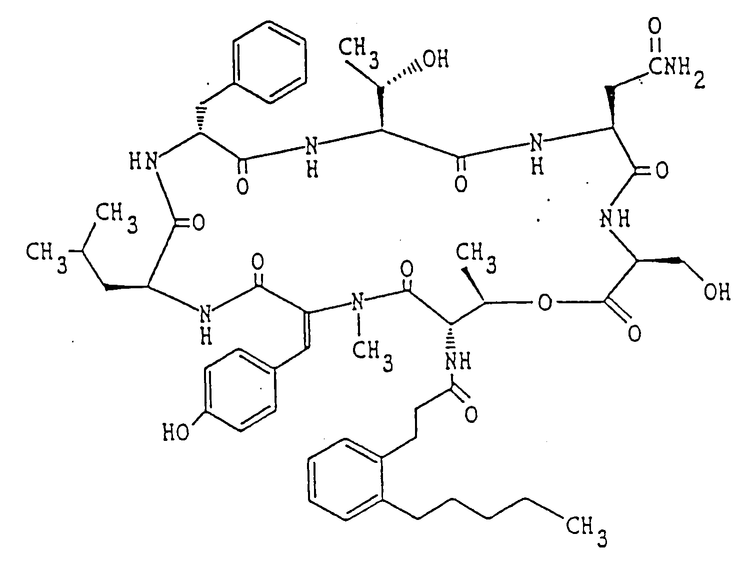

4. Verwendung eines Peptidderivates nach Anspruch 1 oder eines pharmazeutisch verträglichen Salzes davon für die Herstellung eines Medikamentes zur Prävention und/oder Behandlung von Arthritis.

## Revendications

1. Emploi d'un dérivé de peptide répondant à la formule générale : 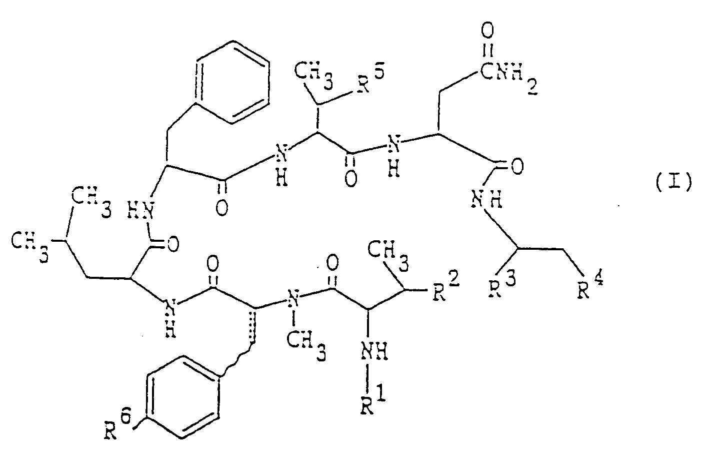 dans laquelle :
R¹ est un atome d'hydrogène, un groupe phényl(alcoxy en C₁-C₆)carbonyle, alcanoyle en C₁-C₆, alcanoyle en C₁₅-C₂₀, benzoyle, thiényl(alcanoyle en Ci-C₆), phényl(alcénoyle en C₃-C₆) substitué par un groupe alcényle en C₂-C₆, ou un groupe phényl(alcanoyle en Ci-C₆) substitué par un groupe alkyle en C₁-C₆;
R² est un groupe hydroxy et
R³ est un groupe carboxy ou un groupe (alcoxy en C₁-C₆)carbonyle, ou
R² et R³ sont liés ensemble pour représenter un groupe de la formule : 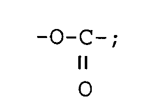
R⁴ est un groupe hydroxy, phényl(alcoxy en C₁-C₆) ou alcanoyloxy en C₁-C₆;
R⁵ est un groupe hydroxy, phényl(alcoxy en C₁-C₆) ou alcanoyloxy en C₁-C₆;
R⁶ est un groupe hydroxy, alcoxy en C₁-C₆, phényl(alcoxy en Ci -C₆ ) ou alcanoyloxy en C₁-C₆; et est une liaison simple ou une liaison double,
ou un de ses sels pharmaceutiquement acceptables pour la fabrication d'un médicament destiné à éviter et/ou à traiter l'inflammation.

2. Utilisation selon la revendication 1, dans laquelle le dérivé de peptide est le composé (I), dans lequel :
R¹ est un groupe 3-(2-pentylphényl)-propanoyle,
R² et R³ sont liés ensemble pour représenter un groupe de la formule : 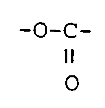
R⁴ est un groupe hydroxy,
R⁵ est un groupe hydroxy,
R⁶ est un groupe hydroxy, et est une liaison double,
ou un de ses sels pharmaceutiquement acceptables.

3. Utilisation selon la revendication 2, dans laquelle le dérivé de peptide est un composé répondant à la formule :

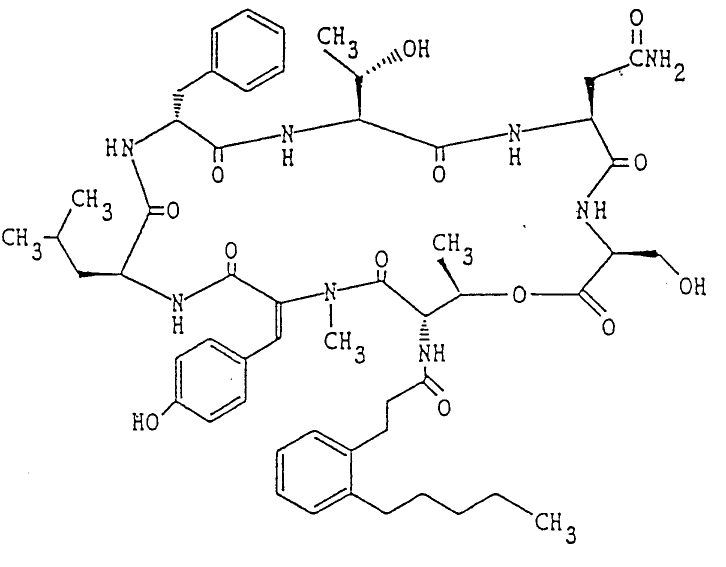

4. Utilisation d'un dérivé de peptide selon la revendication 1, ou d'un de ses sels pharmaceutiquement acceptables pour la fabrication d'un médicament destiné à la prévention et/ou au traitement de l'arthrite.
